# EUROPÄISCHE PATENTSCHRIFT

(11) **EP 2 835 139 B1**
(45) Veröffentlichungstag und Bekanntmachung des Hinweises auf die Patenterteilung: **05.06.2019**
(21) Anmeldenummer: 14174643.8
(22) Anmeldetag: 27.06.2014
(51) Int. Cl.: A61L 27/04, A61L 27/54, A61L 27/58, A61L 31/02, A61L 31/14, A61L 31/16, C22B 23/00

(54) **Bioresorbierbare Werkstoffverbunde, enthaltend Magnesium und/oder Magnesiumlegierungen sowie Implantate aus diesen Verbunden**
Bioresorbable material composites, containing magnesium and/or magnesium alloys and implants made from these composites
Composites de matière active bio-résorbables, contenant du magnésium et/ou alliages de magnésium et implants fabriqués à partir de ces composites

(30) Priorität: 26.07.2013 DE 102013214636
(43) Veröffentlichungstag der Anmeldung: 11.02.2015
(73) Patentinhaber: Heraeus Medical GmbH, 61273 Wehrheim (DE); Medizinische Universität Graz, 8010 Graz (AT)
(72) Erfinder: Kühn, Klaus-Dieter, 34587 Felsberg (DE); Weinberg, Annelie, 48465 Schüttorf (AT); Uggowitzer, Peter, 8913 Ottenbach (CH); Vogt, Sebastian, 99092 Erfurt (DE); Löffler, Jörg, 8606 Greifensee (CH)
(74) Vertreter: Heraeus IP

(56) Entgegenhaltungen:
- WO-A2-2012/003502
- WO-A2-2013/052791
- US-A1- 2009 081 313
- MEISAM SALAHSHOOR; YUEBIN GUO: "Biodegradable Orthopedic Magnesium-Calcium (MgCa) Alloys, Processing, and Corrosion Performance", MATERIALS, Bd. 5, 2012, Seiten 135-155, XP002733581,

## Beschreibung

Die vorliegende Erfindung betrifft bioresorbierbare Werkstoffverbunde, enthaltend Magnesium und Magnesiumlegierungen, insbesondere Magnesium-Calcium-Legierungen sowie Implantate aus diesen Verbunden.

Im Gegensatz zu Polymermaterialien, die in der Regel relativ schlechte mechanische Eigenschaften zeigen, und Keramikmaterialien, welche eine geringe Duktilität und Zähigkeit besitzen, bieten metallische Werkstoffe insgesamt gute mechanische Eigenschaften, so dass in vivo abbaubare Werkstoffe auf Metallbasis vermehrt für medizinische Anwendungen in Frage kommen.

Neben anderen Metallen finden insbesondere auch Magnesium und Magnesiumlegierungen Anwendung. Diese Werkstoffe sind insofern ideal, als ihre elastischen Eigenschaften denen von Knochen entsprechen und somit bei mechanischer Belastung keine Spannung zwischen den aus diesen Werkstoffen hergestellten Implantaten und vorhandenem Knochen entsteht.

In Materials 2012, 5, 135-155 (www.mdpi.com/journal/materials, open access), "Biodegradable Orthopedic Magnesium-Calcium (MgCa) Alloys, Processing, and Corrosion Performance" von Meisam Salahshoor und Yuebin Guo werden Magnesium-Calcium-Legierungen und ihre Eigenschaften für medizinische Anwendungen beschrieben. Wie auch hier dargelegt wird, besteht ein Problem bei der Verwendung von Magnesium oder Magnesiumlegierungen als bioresorbierbare Implantate zum Einen in einer hohen Abbaugeschwindigkeit und zum Anderen darin, dass beim Abbau von Magnesium Wasserstoff entsteht. Aufgrund der hohen Abbaugeschwindigkeit und Bildung großer Mengen von Wasserstoff, welche nicht in dieser Geschwindigkeit vom Körper aufgenommen werden können, besteht die Gefahr der Bildung subkutaner Gastaschen. Daneben führt die schnelle Korrosion von Magnesium zu einem zu frühen Verlust mechanischer Integrität.

US2009/0081313 A1 beschreibt eine Magnesiumlegierung, die durch Biokompatibiltität, mechanische Eigenschaften und eine Abbaugeschwindigkeit gekennzeichnet ist, welche für medizinische Anwendungen vorteilhaft sind. Die Legierung enthält vom 1,5 bis 5 Gew.-% Neodym und 0,1 bis 4 Gew.-% Yttrium.

Aufgabe der vorliegenden Erfindung ist es daher, einen Werkstoff bereitzustellen, welcher die vorteilhaften Eigenschaften von Magnesium und dessen Legierungen zeigt, gleichzeitig dessen Nachteile aber kompensiert.

Überraschend hat sich gezeigt, dass Werkstoffverbünde aus reinem Magnesium oder Magnesiumlegierungen, welche einen oder mehrere antiinfektive Wirkstoffe mit einer Löslichkeit in Wasser bei Zimmertemperatur (25°C) von weniger als 10 mg/l aufweisen, diese Aufgabe erfüllen.

Erfindungsgemäßes Magnesium oder eine erfindungsgemäße Magnesiumlegierung wird bevorzugt aus hochreinem Magnesium als Ausgangsstoff hergestellt. Hierzu wird z.B. im Handel erhältliches Magnesium konventioneller Reinheit (99,8%; z.B. erhältlich von AMAG Austria Metall AG, A) mit Hilfe von Vakuumdestillation auf eine Reinheit von mehr als 99,99% gebracht. Anstelle des reinen Magnesiums können auch Magnesiumlegierungen, bevorzugt Magnesium-Calcium-Legierungen verwendet werden, die unter Schutzgas aus hochreinem Magnesium und einem weiteren biokompatiblen Metall (z.B. Calcium, Zink) nach bekannten Verfahren hergestellt werden. Anschließende Anlass- und Extrusionsschritte dienen der Verbesserung der mechanischen Eigenschaften der Magnesium-Calcium-Legierungen oder Magnesium-Calcium-X-Legierungen, wobei X ein weiteres biokompatibles und biodegradierbares Element ist, durch gezielte Veränderung von deren Mikrostruktur. Andere nach dem Stand der Technik bekannte Formgebungsverfahren sind ebenfalls möglich, solange die Reinheit der Werkstoffe erhalten bleibt.

Bevorzugt besteht der Magnesiumpartner des Werkstoffverbundes aus Reinmagnesium mit einem Gesamtgehalt an Verunreinigungen von kleiner 0,05 Gew.-%, bevorzugt kleiner 0,01 Gew.-% oder aus einer Magnesium-Calcium-Legierung mit einem Calciumgehalt im Bereich von Spuren (d.h. von 1 ppm, bevorzugt von 10 ppm) bis zum Eutektikum der Legierung (d.h. 16,2 Gew.-%). Besonders bevorzugt sind Magnesium-Calcium-Legierungen des Strukturtyps C14 (Laves-Phase, Stöchiometrie Mg2Ca). Diese werden durch Wärmebehandlung unter 400°C gebildet und dienen der Kornfeinung und somit der Verbesserung der mechanischen Eigenschaften. Da Calcium unedler als Magnesium ist, wirkt diese Phase nicht kathodisch, sondern wird in wässriger Lösung (z.B. in Körperflüssigkeiten, wie Blut) anodisch aufgelöst und lässt somit die "ungestörte" Magnesiumoberfläche zurück. Besonders bevorzugt liegt der Calciumgehalt der Magnesiumlegierung zwischen 0,1 Gew.-% und 1 Gew.-%, insbesondere zwischen 0,2 Gew.-% und 0,6 Gew.-%. In allen Legierungen liegt der Gesamtgehalt an biologisch fragwürdigen Verunreinigungselementen (z.B. Seltene Erden, Aluminium, Kupfer) unter 50 ppm, bevorzugt unter 10 ppm. Biologisch fragwürdige Verunreinigungselemente im Sinne der Erfindung sind Elemente, die toxische Wirkung auf den Körper haben (z.B. Be, Pb, Hg, As, Cr, Cu), aller-gische Reaktionen auslösen können (z.B. Ni, Co, Cr), krebserregende Wirkung zeigen (z.B. Cr-Verbindungen), oder in Verdacht stehen, Krankheiten auszulösen (z.B. AI).

Der erfindungsgemäße Wirkstoffverbund weist einen in Wasser schwer löslichen organisch antiinfektiven Wirkstoff auf. Das bedeutet, dass der antiinfektive Wirkstoff bei 25 °C eine Löslichkeit von weniger als 10 g pro Liter hat. Bevorzugt hat der antiinfektive Wirkstoff eine Löslichkeit in Wasser von weniger als 2 g pro Liter. Bedingt durch die geringe Löslichkeit des Wirkstoffes in Wasser ergibt sich eine verzögerte Lösung des Wirkstoffes in vivo. Dadurch wird der Wirkstoff langsam und über einen längeren Zeitraum in relativ geringen Konzentrationen in den Organismus freigesetzt. Gleichzeitig wird die Resorption der Magnesium-Verbundkomponente verzögert, die Bildung von Wasserstoff erfolgt über einen längeren Zeitraum.

Grundsätzlich ist jeder antiinfektive Wirkstoff geeignet, solange er bei 25 °C eine Löslichkeit in Wasser von weniger als 10 g pro Liter Wasser aufweist. Der antiinfektive Wirkstoff kann aus der Gruppe von gegen Bakterien, Pilze und Viren wirksamen Substanzen oder einer Mischung davon ausgewählt sein. Erfindungsgemäß zu verwendende antiinfektive Wirkstoffe richten sich gegen insbesondere für den Menschen pathologische Keime, beispielsweise gegen grampositive Keime wie Staphylococcus aureus, Staphylococcus albus und Streptococcus, gramnegative Keime wie Escherichia coli, Bacterium proteus, Pseudomonas, Enterococcus und Klebstella.

Beispiele für geeignete Antibiotika sind Antibiotika aus der Gruppe der Aminoglycoside, der Lincosamide, der Glycopetide, der Polymyxine, der Oxazolidinone. Erfindungsgemäß können Antibiotika in jeder Form vorliegen, in der das Antibiotikum antiinfektive Wirksamkeit entfaltet oder die Freisetzung einer Verbindung mit antiinfektiver Wirkung ermöglicht. Somit fallen unter den Begriff Antibiotika auch Antibiotikasalze oder Antibiotikaester sowie die entsprechenden hydratisierten Formen der Antibiotika, Antibiotikasalze oder Antibiotikaester. Bevorzugt werden schwerlösliche Fettsäuresalze der Aminoglycoside eingesetzt. Beispiele hierfür sind Gentamicinmyristat, Gentamicinpalmitat, Gentamicinstearat, Tobramycinmyristat, Tobramycinpalmitat, Tobramycinstearat, Amikacinmyristat, Amikacinpalmitat, Amikacinstearat, Vancomycinpalmitat, Vancomycinstearat, Ramoplaninpalmitat, Ramoplaninstearat, Levofloxacinpalmitat, Levofloxacinstearat, Ofloxacinpalmitat, Ofloxacinstearat, Moxifloxacinpalmitat, Moxifloxacinstearat, Clindamycinpalmitat und Clindamycinstearat. Unter den Begriffen Palmitat, Stearat und Myristat werden die Antibiotika-Salze der Palmitinsäure, der Stearinsäure und der Myristinsäure verstanden. Dabei ist das bevorzugte Stoffmengenverhältnis von protonierter Aminogruppe zu Fettsäure-Anion gleich 1. Es ist jedoch auch möglich, dass nur ein Teil der protonierten Aminogruppen Fettsäure-Anionen als Gegenionen besitzen. So können zum Beispiel Gentamicinpentakispalmitat, Gentamicintetrakispalmitat oder auch Gentamicintripalmiat als in Wasser gering lösliche Antibiotika-Salze verwendet werden.

Ferner sind kationische und/oder anionische Antiseptika geeignet, wobei Chlorhexidin, Octenidin, Dequaliniumchlorid, Polyhexanid und oligomere Biguanide besonders bevorzugt sind. Weiterhin kann ein Antiseptikum aus den Gruppen der Wachstumsfaktoren und insbesondere BMP-2, BMP-7 und davon abgeleiteten Proteine, di-Neoangiogenese auslösende Proteine, Steroidhormone, Bisphosphonate und Antiphlogistika eingestezt werden.

Die Erfindung betrifft ferner medizinische Implantate, die aus dem Werkstoffverbund hergestellt werden. Dabei wird zunächst ein geometrischer Körper aus der Magnesiumkomponente hergestellt werden, woraufhin anschließend der antiinfektive Wirkstoff auf der Oberfläche des geometrischen Körpers aufgebracht wird. Außerdem können Magnesiumkomponente und antiinfektiver Wirkstoff auch in Verbünden, die zum Beispiel aus mehreren Schichten aufgebaut sind, vorliegen.

Der antiinfektive Wirkstoff liegt in einer Konzentration von 0,1 bis 100 mg, insbesondere von 1 bis 10 mg pro Gramm resorbierbares Implantatmaterial vor, je nachdem wie der eingesetzte Wirkstoff konzentriert ist bzw. welches Beschichtungs- bzw. Beladungsverfahren verwendet wird, um die geometrischen Magnesiumkörper auszurüsten.

Die Beschichtung liegt in einer Dicke von 0,001 bis 1 mm, insbesondere von 0,01 bis 0,5 mm vor. Die Oberfläche des Magnesiumkörpers kann teilweise oder vollständig von dem antiinfektiven Wirkstoff bedeckt sein, bevorzugt ist die gesamte Oberfläche der Verbundkomponente bedeckt.

Das Beschichten kann nach üblichen und den Fachleuten bekannten Beschichtungsverfahren vorgenommen werden, wie beispielsweise Tauch- oder Sprühverfahren oder die Werkstoffkörper können betropft werden. Zu diesem Zweck kann der Wirkstoff in Form einer Lösung oder einer Suspension aufgebracht werden. Beispiele für geeignete Lösemittel umfassen z.B. Alkohole, wie Methanol, Ethanol, etc. Feste Stoffe können auch z.B. durch Aufschmelzen an Oberflächen angebracht werden.

Weiterhin kann die Beschichtung bzw. Beladung auch durch Wirkstoffe in fester Form vorgenommen werden.

Bei dem geometrischen Körper handelt es sich um Platten oder Drähte. Überraschend hat sich herausgestellt, dass sich die vorliegende Erfindung besonders gut für filigrane Implantate, wie Drähte, Netze, Gewebe etc. eignet.

Bevorzugt wird der Werkstoffverbund für Körper mit gestreckter Geometrie verwendet, wobei ein Körper aus der Magnesiumkomponente ein Achsverhältnis von > 500, bevorzugt >1000 aufweist. Hierbei bildet der Körper einen Draht. Ein solcher Draht hat einen Durchmesser von kleiner 500 µm, bevorzugt kleiner 200 µm und besonders bevorzugt kleiner 100 µm.

Die Drähte können allein oder zu mehreren in beliebiger Geometrie angeordnet sein. So können beispielsweise zwei oder mehrere Drähte wendelförmig ein röhrenförmiges Flächengebilde ausbilden. Dabei sind bevorzugt zumindest zwei Drähte gegenläufig übereinander mit gleicher oder unterschiedlicher Ganghöhe angeordnet. Auf diese Weise geflochtene Implantate, insbesondere Stents können durch die Wahl der Drähte sehr fein eingestellt werden.

Dabei kann ein so gebildetes röhrenförmiges Flächengebilde in der Längsachse durch plastische Verformung mechanisch verzahnt sein. Die Verzahnung erfolgt bevorzugt durch mindestens eine partielle Verformung des Drahtes nach innen oder nach außen, wobei die Krümmung der Verzahnung klein gegenüber der Kreisform des röhrenförmigen Flächengebildes ist.

Im Implantat können die Werkstoffverbundkomponenten auch in Schichten angeordnet sein. Dabei weisen beide Verbundkomponenten eine flächenförmige Geometrie auf. Die Komponenten können in diesem Fall zu einem Parallelverbund geschichtet werden und ggfs. an den Enden durch Kraftverbund, Stoffverbund oder Formverbund zusammengefügt werden. Dabei können einzelne Schichten der Magnesiumkomponente eine Perforierung aufweisen.

Eine einzelne Schicht der Magnesiumkomponente im Werkstoffverbund hat eine Dicke kleiner 500 µm, bevorzugt kleiner 200 µm und besonders bevorzugt kleiner 100 µm.

Im Folgenden wird die Erfindung und ihre Ausführungsformen anhand von Beispielen näher erläutert.

### Beispiel A: Herstellung einer Verbundkomponente aus Magnesium hoher Reinheit zur Erzeugung eines Verbundkörpers in flacher Geometrie

Magnesium mit konventioneller Reinheit (99.8%; Quelle AMAG Austria Metall AG, A) wurde mit Hilfe von Vakuumdestillation (Testanlage der ETH Zürich) auf eine Reinheit von 99.998% gebracht. Danach wurde der bei der Destillation entstandene Magnesiumbolzen mit 55 mm Durchmesser und 110 mm Länge mechanisch auf einen Durchmesser von 50 mm abgedreht und in einem Widerstandsofen auf 300 °C erhitzt. Mit Hilfe einer Extrusionsanlage (Fa. Müller-Engineering, Friedberg, D) wurde der vorgewärmte Bolzen zu einem Rundprofil mit Durchmesser 6 mm verpresst. In einem weiteren Schritt wurden aus dem Rundprofil in Längsrichtung Plättchen der Geometrie Länge 10 mm, Breite 5 mm Dicke 0.25 mm herausgeschnitten. Dies erfolgte mit Hilfe von Drahterosion.

### Beispiel B: Herstellung einer Verbundkomponente aus einer Magnesium-Calcium-Legierung zur Erzeugung eines Verbundkörpers in gestreckter Geometrie

Magnesium mit hoher Reinheit (99.95%; Quelle Alfa Aesar; Karlsruhe, D) wurde in einem Widerstandsofen unter Stickstoff mit SF6-Zusatz mit 0.3 Gewichts-% Calcium auflegiert. Der so entstandene Gussbolzen mit 55 mm Durchmesser und 110 mm Länge wurde mechanisch auf einen Durchmesser von 50 mm abgedreht und in einem Widerstandsofen auf 325°C erhitzt. Mit Hilfe einer Extrusionsanlage (Fa. Müller-Engineering, Friedberg, D) wurde der vorgewärmte Bolzen zu einem Rundprofil mit Durchmesser 6 mm verpresst. Weitere Verformung des Profils von 6 mm auf 3 mm Durchmesser erfolgte bei 300 °C auf einer Rundknet-Hämmermaschine (Fa. Bock, Lüdenscheid, D). In zwölf bei Raumtemperatur durchgeführten Ziehschritten an einer Goldschmied-Ziehmatrize mit jeweils 10 Minuten Zwischenglühung bei 300 °C wurde ein Feindraht mit 0.2 mm Durchmesser hergestellt.

### Vergleichsbeispiel 1 und Beispiele 2-5: Herstellung von Verbundkörpern durch Beschichtung der in den Beispielen A und B hergestellten Verbundkomponenten mit Gentamicinpalmitat

Es wurde zunächst einer 4%ige methanolischen Gentamicinpalmitat-Lösung hergestellt. Dabei wurden 0,4 g Gentamicinpalmitat in 9,6 g Amin-freiem Methanol unter Rühren bei Raumtemperatur gelöst. Es entsteht eine klare, schwach gelbliche Lösung.

Als Verbundkomponente wurden die Plättchen (1,0 x 0,5 cm) (Beispiele 2 und 3) aus Beispiel A und Drähte (10 cm lang, Beispiele 4 und 5) aus Beispiel B verwendet, welche zunächst auf 90°C temperiert und anschließend mit methanolischer Gentamicinpalmitat-Lösung betropft wurden. Das Methanol verdampfte sofort und das Gentamicinpalmitat bildete eine farblose bis schwach trübe Beschichtung auf der Probekörperoberfläche aus.

In Tabelle 1 ist die Masse der Werkstoffverbunde vor und nach der Beschichtung gezeigt. Vergleichsbeispiel 1 zeigt eine unbeschichtete Verbundkomponente.

### Beispiele 6-9 und Vergleichsbeispiel 10: Herstellung von Verbundkörpern durch Beschichtung der in den Beispielen A und B hergestellten Verbundkomponenten mit Octenidin/Laurinsäure-Lösung

Es wurde zunächst ein Octenidin/Laurinsäure-Gemisch hergestellt. Dabei wurden 10,0 g Laurinsäure in einem Becherglas bei 80 °C aufgeschmolzen. Anschließend wurden 15,0 g Octenidinhydrochlorid zugegeben. Das Gemisch wurde unter Rühren homogenisiert. Danach wurde das Gemisch auf Raumtemperatur abgekühlt.

Anschließend wurde eine Octenidin/Laurinsäure-Lösung hergestellt, wobei 0,5 g des Octenidin/Laurinsäure-Gemischs in 25,0 g Ethanol bei Raumtemperatur gelöst wurden.

Als Verbundkomponente wurden die Plättchen (1,0 x 0,5 cm) (Beispiele 2 und 3) aus Beispiel A und Drähte (10 cm lang, Beispiele 4 und 5) aus Beispiel B verwendet, welche zunächst auf 90°C temperiert und anschließend mit ethanolischer Octenidin/Laurinsäure-Lösung betropft wurden. Das Lösungsmittel verdampfte sofort und es bildete sich eine farblose bis weiß-trübe Beschichtung aus.

In Tabelle 1 ist die Masse der Werkstoffverbunde vor und nach der Beschichtung gezeigt. Vergleichsbeispiel 10 zeigt eine unbeschichtete Verbundkomponente.

**Tab. 1: Übersicht über die beschichteten Probekörper**

| Beispiel | Material | Masse vor der Beschichtung [mg] | Masse nach der Beschichtung [mg] | Beschichtung [mg] | Material |
|---|---|---|---|---|---|
| 1 | Plättchen | - | - | Vergleich | - |
| 2 | Plättchen | 23,4 | 23,9 | 0,5 | Gentamicinpalmitat |
| 3 | Plättchen | 22,3 | 23,1 | 0,8 | Gentamicinpalmitat |
| 4 | Draht | 6,3 | 7,0 | 0,7 | Gentamicinpalmitat |
| 5 | Draht | 6,0 | 8,3 | 2,3 | Gentamicinpalmitat |
| 6 | Plättchen | 31,4 | 31,8 | 0,4 | Octenidin/Laurinsäure |
| 7 | Plättchen | 23,0 | 45,7 | 22,7 | Octenidin/Laurinsäure |
| 8 | Draht | 5,9 | 6,2 | 0,3 | Octenidin/Laurinsäure |
| 9 | Draht | 5,9 | 14,3 | 8,4 | Octenidin/Laurinsäure |
| 10 | Draht | - | - | Vergleich | - |

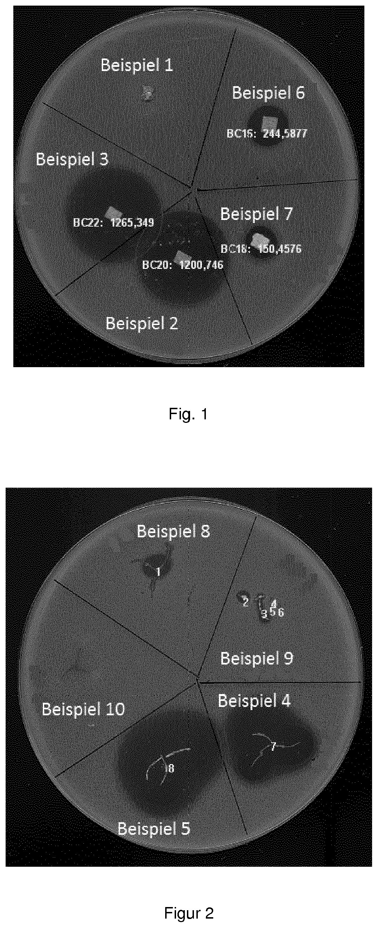

Die in den Beispielen und Vergleichsbeispielen hergestellten Werkstoffverbunde wurden einem Agardiffusionstest (Hemmhoftest) unterzogen, wobei steriler Nähragar I mit Bacillus subtilissporen ATCC 6633 beimpft wurden. Der beimpfte Nähragar wurde daraufhin in sterile Petrischalen gegossen. Nach Erkalten des Nähragars wurden die Werkstoffverbunde auf die Oberfläche des Nähragars aufgelegt. Nachdem die Probenkörper 24 Stunden bei 37 °C bebrütet wurden, wurden die Hemmhöfe mit einem üblichen Scanner eingescannt. Die Ergebnisse sind in Tabelle 2 aufgeführt. Figuren 1 und 2 zeigen die gescannten Bilder, wobei Figur 1 die Hemmhofbildung bei Beispielen 1, 2,3, 6 und 7 zeigt und Figur 2 die Hemmhofbildung bei Beispielen 4, 5, 8, 9 und 10.

**Tab.2: Übersicht über die Ergebnisse des mikrobiellen Agardiffusionstest**

| Beispiel | Material | Beschichtung [mg] | Material | Ergebnis |
|---|---|---|---|---|
| 1 | Plättchen | Vergleich | - | Wachstum |
| 2 | Plättchen | 0,5 | Gentamicinpalmitat | Hemmung |
| 3 | Plättchen | 0,8 | Gentamicinpalmitat | Hemmung |
| 4 | Draht | 0,7 | Gentamicinpalmitat | Hemmung |
| 5 | Draht | 2,3 | Gentamicinpalmitat | Hemmung |
| 6 | Plättchen | 0,4 | Octenidin/Laurinsäure | Hemmung |
| 7 | Plättchen | 22,7 | Octenidin/Laurinsäure | Hemmung |
| 8 | Draht | 0,3 | Octenidin/Laurinsäure | Hemmung |
| 9 | Draht | 8,4 | Octenidin/Laurinsäure | Hemmung |
| 10 | Draht | Vergleich | - | Wachstum |

## Patentansprüche

1. Werkstoffverbund, enthaltend mindestens eine Magnesiumkomponente aus Magnesium oder einer Magnesiumlegierung, und mindestens einen organischen antiinfektiven Wirkstoff, wobei der antiinfektive Wirkstoff eine Löslichkeit in Wasser bei Raumtemperatur von kleiner 10 Gramm pro Liter aufweist, **gekennzeichnet durch** einen geometrischen Körper aus einer Magnesiumkomponente, wobei der Gesamtgehalt an seltenen Erden in der Magnesiumkomponente unter 50 ppm liegt, wobei die Oberfläche eines Magnesiumkörpers aus der Magnesiumkomponente teilweise oder vollständig durch das Antiinfektivum bedeckt ist, wobei die Dicke der Beschichtung zwischen 0,001 bis 1 mm ist, wobei das Antiinfektivum in einer Konzentration von 0,1 bis 100 mg pro Gramm absorbierbarem Implantationsmaterial vorliegt und wobei die Magnesiumkomponente des Verbundes einen Draht mit einem Durchmesser von weniger als 500 µm bildet oder beide Verbundkomponenten eine flächenförmige Geometrie aufweisen, die zu einem Parallelverbund geschichtet sind, wobei eine einzelne Schicht der Magnesiumkomponente eine Dicke kleiner 500 µm hat.

2. Werkstoffverbund nach Anspruch 1, **dadurch gekennzeichnet, dass** die Magnesiumkomponente aus Reinmagnesium oder aus einer Magnesium-Calcium-Legierung oder aus einer Magnesium-Calcium-X-Legierung mit einem Calciumgehalt im Bereich von 1 ppm bis 16,2 Gew.-% besteht, wobei X ein weiteres biodegradierbares Element ist.

3. Werkstoffverbund nach Anspruch 2, **dadurch gekennzeichnet, dass** die Magnesiumkomponente eine Magnesium-Calcium-Legierung ist mit einem Calciumgehalt von 0,1 bis 1,0 Gew.-%.

4. Werkstoffverbund nach einem der Ansprüche 1, 2 oder 3, **dadurch gekennzeichnet, dass** der Gesamtgehalt biologisch fragwürdiger Verunreinigungselemente der Magnesiumkomponente unter 50 ppm liegt, bevorzugt unter 10 ppm liegt.

5. Werkstoffverbund nach einem der vorhergehenden Ansprüche, **dadurch gekennzeichnet, dass** der antiinfektive Wirkstoff bei 25°C eine Löslichkeit von < 2 g/L Wasser aufweist.

6. Werkstoffverbund nach einem der vorhergehenden Ansprüche, **dadurch gekennzeichnet, dass** der antiinfektive Wirkstoff mindestens ein Antibiotikum enthält, das aus der Gruppe der Aminoglycosid-Antibiotika, der Lincosamid-Antibiotika, der Glycopetid-Antibiotika, der Polymyxin-Antibiotika, der Oxazolidinone-Antibiotika ausgewählt ist.

7. Werkstoffverbund nach Anspruch 6, **dadurch gekennzeichnet, dass** das Antibiotikum ein Fettsäuresalz der Aminoglycosid-Antibiotika ist.

8. Werkstoffverbund nach einem der Ansprüche 1 bis 5, **dadurch gekennzeichnet, dass** der antiinfektive Wirkstoff mindestens ein Antiseptikum enthält, welches aus der Gruppe ausgewählt ist, die aus kationischen und anionischen Antiseptika und Wachstumsfaktoren besteht.

9. Werkstoffverbund nach Anspruch 8, **dadurch gekennzeichnet, dass** das Antiseptikum aus der Gruppe ausgewählt ist, die aus Chlorhexidin, Octenidin, Dequaliniumchlorid, Polyhexanid, oligomeren Biguaniden, BMP-2, BMP-7 und davon abgeleiteten Proteinen, di-Neoangiogenese auslösenden Proteinen, Steroidhormonen, Bisphosphonaten und Antiphlogistika besteht.

10. Implantat aufweisend einen Werkstoffverbund nach einem der Ansprüche 1 bis 9, **dadurch gekennzeichnet, dass** mindestens eine Magnesiumkomponente des Werkstoffverbunds eine gestreckte Geometrie mit einem Verhältnis von Länge zu Breite von > 1000 aufweist.

11. Implantat nach Anspruch 10, **dadurch gekennzeichnet, dass** jede Magnesiumkomponente einen Draht bildet, welcher in Form einer Helix gewickelt ist, derart dass ein röhrenförmiges Flächengebilde entsteht.

12. Implantat nach Anspruch 11, **gekennzeichnet durch** mindestens zwei Drähte, die gegenläufig mit gleicher oder unterschiedlicher Ganghöhe gewickelt sind, wobei die Drähte partiell nach innen oder außen verformt sind, dass die Verformungen Vorsprünge und Vertiefungen in der Mantelfläche des röhrenförmigen Flächengebildes bilden, derart, dass das röhrenförmige Flächengebilde in der Längsachse mechanisch verzahnt ist.

13. Implantat nach Anspruch 11 oder 12, **dadurch gekennzeichnet, dass** der Draht einen Durchmesser von kleiner 200 µm und bevorzugt kleiner 100 µm aufweist.

14. Implantat aufweisend einen Werkstoffverbund nach einem der Ansprüche 1 bis 9, **dadurch gekennzeichnet, dass** beide Verbundkomponenten eine flächenförmige Geometrie aufweisen, die zu einem Parallelverbund geschichtet werden, und die einzelnen Schichten an ihren Außenkanten durch Kraftverbund, Stoffverbund oder Formverbund aneinander fixiert sind..

15. Implantat nach Anspruch 14, **dadurch gekennzeichnet, dass** einzelne oder alle Schichten der Magnesiumkomponenten eine Perforierung aufweisen.

16. Implantat nach einem der vorangehenden Ansprüche, **dadurch gekennzeichnet, dass** eine einzelne Schicht der Magnesiumkomponente imWerkstoffverbund eine Dicke kleiner 200 µm und bevorzugt kleiner 100 µm aufweist.

## Claims

1. A composite containing at least one magnesium component consisting of magnesium or a magnesium alloy and at least one organic anti-infective agent, wherein the anti-infective agent has a solubility in water of less than 10 grammes per litre at room temperature, **characterised by** a geometrical solid consisting of a magnesium component, wherein the total content of rare earths in the magnesium component is less than 50 ppm, wherein the surface of a magnesium solid consisting of the magnesium component is partially or completely covered with the anti-infective agent, wherein the thickness of the coating is between 0.001 to 1 mm, wherein the anti-infective agent is provided in a concentration of 0.1 to 100 mg per gramme of absorbable implant material and wherein the magnesium component of the composite forms a wire having a diameter of less than 500 µm or both composite components have a planar geometry and are layered to obtain a parallel composite, wherein an individual layer of the magnesium component has a thickness of less than 500 µm.

2. The composite according to claim 1, **characterised in that** the magnesium component consists of pure magnesium or of a magnesium-calcium alloy or of a magnesium-calcium-X alloy having a calcium content ranging from 1 ppm to 16.2 % by weight, wherein X is a further biodegradable element.

3. The composite according to claim 2, **characterised in that** the magnesium component is a magnesium-calcium alloy having a calcium content of 0.1 to 1.0 % by weight.

4. The composite according to any one of claims 1, 2 or 3, **characterised in that** the total content of biologically questionable contamination elements of the magnesium component is less than 50 ppm, preferably less than 10 ppm.

5. The composite according to any one of the preceding claims, **characterised in that** the anti-infective agent has a solubility of < 2 g/L water at 25 °C.

6. The composite according to any one of the preceding claims, **characterised in that** the anti-infective agent contains at least one antibiotic which is selected from the group of aminoglycoside antibiotics, lincosamide antibiotics, glycopeptide antibiotics, polymyxin antibiotics, oxazolidinone antibiotics.

7. The composite according to claim 6, **characterised in that** the antibiotic is a fatty acid salt of the aminoglycoside antibiotics.

8. The composite according to any one of claims 1 to 5, **characterised in that** the anti-infective agent contains at least one antiseptic agent which is selected from the group consisting of cationic and anionic antiseptic agents and growth factors.

9. The composite according to claim 8, **characterised in that** the antiseptic agent is selected from the group consisting of chlorhexidine, octenidine dihydrochloride, dequalinium chloride, polyhexanide, oligomeric biguanides, BMP-2, BMP-7 and proteins derived therefrom, proteins causing di-neoangio-genesis, steroid hormones, bisphosphonates and antiphlogistics.

10. An implant having a composite according to any one of claims 1 to 9, **characterised in that** at least one magnesium component of the composite has a stretched geometry with a ratio of length to width of > 1000.

11. The implant according to claim 10, **characterised in that** each magnesium component forms a wire which is wound in the form of a helix such that a tubular planar structure is being generated.

12. The implant according to claim 11, **characterised by** at least two wires which are wound in opposite directions with the same pitch or a different pitch, wherein the wires are partially deformed inwards or outwards, so that the deformations form projections or recesses in the curved surface area of the tubular planar structure, such that the tubular planar structure is mechanically toothed along the longitudinal axis.

13. The implant according to claim 11 or 12, **characterised in that** the wire has a diameter of less than 200 µm and, preferably, of less than 100 µm.

14. An implant having a composite according to any one of claims 1 to 9, **characterised in that** both composite components have a planar geometry and are layered to obtain a parallel composite and that the individual layers are fixed to each other at their outer edges by a force-fit composite, a firmly bonded composite or a form-fit composite.

15. The implant according to claim 14, **characterised in that** individual layers or all layers of the magnesium components have a perforation.

16. The implant according to any one of the preceding claims, **characterised in that** an individual layer of the magnesium component in the composite has a thickness of less than 200 µm and, preferably, of less than 100 µm.

## Revendications

1. Composite de matériaux contenant au moins un composant de magnésium en magnésium ou un alliage de magnésium et au moins une substance active anti-infectieuse organique, dans lequel la substance active anti-infectieuse présente une solubilité dans l'eau à température ambiante inférieure à 10 grammes par litre, **caractérisé par** un corps géométrique en un composant de magnésium, dans lequel la teneur totale en terres rares dans le composant de magnésium se situe en dessous de 50 ppm, dans lequel la surface d'un corps de magnésium en le composant de magnésium est partiellement ou entièrement recouverte par l'agent anti-infectieux, dans lequel l'épaisseur de l'enrobage est comprise entre 0,001 et 1 mm, dans lequel l'agent anti-infectieux est présent à une concentration de 0,1 à 100 mg par gramme de matériau d'implantation absorbable et dans lequel le composant de magnésium du composite forme un fil avec un diamètre inférieur à 500 µm ou les deux composants de composite présentent une géométrie planiforme qui sont disposés par couches en un composite parallèle, dans lequel une couche individuelle du composant de magnésium a une épaisseur inférieure à 500 µm.

2. Composite de matériaux selon la revendication 1, **caractérisé en ce que** le composant de magnésium se compose de magnésium pur ou d'un alliage de magnésium-calcium ou d'un alliage de magnésium-calcium-X avec une teneur en calcium dans la région de 1 ppm à 16,2 % en poids, dans lequel X est un autre élément biodégradable.

3. Composite de matériaux selon la revendication 2, **caractérisé en ce que** le composant de magnésium est un alliage de magnésium-calcium avec une teneur en calcium de 0,1 à 1,0 % en poids.

4. Composite de matériaux selon une des revendications 1, 2 ou 3, **caractérisé en ce que** la teneur totale en éléments d'impureté biologiquement douteux du composant de magnésium se situe en dessous de 50 ppm, se situe de préférence en dessous de 10 ppm.

5. Composite de matériaux selon une des revendications précédentes, **caractérisé en ce que** la substance active anti-infectieuse présente à 25°C une solubilité de < 2 g/l d'eau.

6. Composite de matériaux selon une des revendications précédentes, **caractérisé en ce que** la substance active anti-infectieuse contient au moins un antibiotique qui est sélectionné parmi le groupe des antibiotiques d'aminoglycoside, des antibiotiques de lincosamide, des antibiotiques de glycopeptide, des antibiotiques de polymyxine, des antibiotiques d'oxazolidinone.

7. Composite de matériaux selon la revendication 6, **caractérisé en ce que** l'antibiotique est un sel d'acide gras des antibiotiques d'aminoglycoside.

8. Composite de matériaux selon une des revendications 1 à 5, **caractérisé en ce que** la substance active anti-infectieuse contient au moins un antiseptique qui est sélectionné parmi le groupe qui se compose d'antiseptiques cationiques et anioniques et de facteurs de croissance.

9. Composite de matériaux selon la revendication 8, **caractérisé en ce que** l'antiseptique est sélectionné parmi le groupe qui se compose de la chlorhexidine, de l'octénidine, du chlorure de déqualinium, du polyhexanide, de biguanides oligomères, de protéines BMP-2, BMP-7 et dérivées de celles-ci, de protéines déclenchant la di-néoangiogenèse, d'hormones stéroïdiennes, de bisphosphonates et d'anti-inflammatoires.

10. Implant présentant un composite de matériaux selon une des revendications 1 à 9, **caractérisé en ce qu'**au moins un composant de magnésium du composite de matériaux présente une géométrie étirée avec un rapport de longueur sur largeur de > 1000.

11. Implant selon la revendication 10, **caractérisé en ce que** chaque composant de magnésium forme un fil qui est enroulé sous forme d'une hélice de telle sorte qu'une formation plane tubulaire se forme.

12. Implant selon la revendication 11, **caractérisé par** au moins deux fils qui sont enroulés de manière opposée avec un pas identique ou différent, dans lequel les fils sont partiellement déformés vers l'intérieur ou l'extérieur, que les déformations forment des saillies et renfoncements dans la face d'enveloppe de la formation plane tubulaire de telle sorte que la formation plane tubulaire est mécaniquement dentée dans l'axe longitudinal.

13. Implant selon la revendication 11 ou 12, **caractérisé en ce que** le fil présente un diamètre inférieur à 200 µm et de préférence inférieur à 100 µm.

14. Implant présentant un composite de matériaux selon une des revendications 1 à 9, **caractérisé en ce que** les deux composants de composite présentent une géométrie planiforme qui sont disposés par couches en un composite parallèle, et les couches individuelles sont fixées les unes aux autres à leurs arêtes externes par connexion par friction, liaison de matières ou conjugaison de formes.

15. Implant selon la revendication 14, **caractérisé en ce que** des couches individuelles ou toutes les couches des composants de magnésium présentent une perforation.

16. Implant selon une des revendications précédentes, **caractérisé en ce qu'**une couche individuelle du composant de magnésium présente dans le composite de matériaux une épaisseur inférieure à 200 µm et de préférence inférieure à 100 µm.
